(19) Europäisches Patentamt
European Patent Office
Office européen des brevets

(11) EP 1 726 660 A1

(12) # EUROPEAN PATENT APPLICATION
published in accordance with Art. 158(3) EPC

(43) Date of publication:
**29.11.2006 Bulletin 2006/48**

(21) Application number: **05720893.6**

(22) Date of filing: **16.03.2005**

(51) Int Cl.:
***C12Q 1/37*** *(2006.01)* ***C12Q 1/26*** *(2006.01)*
***C12Q 1/28*** *(2006.01)* ***G01N 33/72*** *(2006.01)*

(86) International application number:
**PCT/JP2005/004639**

(87) International publication number:
**WO 2005/087946 (22.09.2005 Gazette 2005/38)**

(84) Designated Contracting States:
**AT BE BG CH CY CZ DE DK EE ES FI FR GB GR HU IE IS IT LI LT LU MC NL PL PT RO SE SI SK TR**

(30) Priority: **17.03.2004 JP 2004076014**

(71) Applicant: **DAIICHI PURE CHEMICALS CO., LTD.**
**Chuo-ku**
**Tokyo 103-0027 (JP)**

(72) Inventors:
- **TANIGUCHI, Yuriko**
**DAIICHI PURE CHEMICALS CO.LTD**
**RYUGASAKI-SHI, Ibaraki 3010852 (JP)**
- **NISHIO, Tomohisa**
**DAIICHI PURE CHEMICALS CO.LTD**
**RYAGASAKI-SHI, Ibaraki 3010852 (JP)**
- **SAITO, Kazunori**
**DAIICHI PURE CHEMICALS CO.LTD**
**RYAGASAKI-SHI, Ibaraki 3010852 (JP)**

(74) Representative: **Hartz, Nikolai**
**Wächtershauser & Hartz**
**Weinstrasse 8**
**80333 München (DE)**

(54) **METHOD OF MEASURING GLYCOPROTEIN**

(57) A method for measuring a glycated protein, a glycated peptide, or a glycated amino acid is provided. In this method, proteolytic activity of the protease is controlled to thereby realize high accuracy of the measurement. Also provided is a reagent used in such a measurement. More specifically, this invention provides a method of measuring a glycatedprotein, aglycatedpeptide, or a glycated amino acid comprising the steps of treating a sample containing the glycated protein with a protease for releasing a glycated peptide or a glycated amino acid; reacting the released glycated peptide or glycated amino acid with corresponding oxidase for generation of hydrogen peroxide; and measuring the resulting hydrogen peroxide with peroxidase and an oxidizable color developing reagent; wherein reaction solution before the reaction with the oxidase is adjusted to a pH of 1 to 5; and a reagent used in such measurement.

EP 1 726 660 A1

## Description

### Technical Field

This invention relates to a method of measuring a glycatedprotein, a glycated peptide, or a glycated amino acid in a sample, and a reagent used in such glycated protein measurement.

### Background Art

A glycated protein is a protein generated by unenzymatic glycation of a protein, namely an Amadori compound generated by the formation of a Schiff base by the binding of the aldehyde group of the sugar and the amino group of the protein and the subsequent Amadori rearrangement. Glycated proteins are found in a wide variety of locations in the living body, and among such glycated proteins, concentration of the glycated proteins present in blood depends on the concentration of the single sugars such as glucose dissolved in blood. Examples of the glycatedprotein include those having a glycated α-amino group at the amino terminal (for example, glycated hemoglobin) and those in which ε-amino group of the lysine in the interior of the protein has been glycated (for example, glycated albumin). Since concentration of the glycated albumin in blood, and ratio of the glycated hemoglobin and the non-glycated hemoglobin in the erythrocyte reflect, average blood glucose level of in the preceding period of certain length, such concentration and ratio are used as an index for diagnosing diabetes, controlling the disease condition, and evaluating therapeutic effects.

A typical method for measuring glycated protein is the one using an enzyme (see, for example, Patent Documents 1 and 2). This enzymatic method comprises the step of pretreatment in which a protease is reacted with the glycated protein in the sample for release of the glycated peptide or glycated amino acid which serves a substrate in the subsequent step; the step in which glycated peptide-specific enzyme or a glycated amino acid-specific enzyme (for example, oxidase) is reacted with the released substrate for generation of a detectable substance (for example, hydrogen peroxide) ; and the step of detecting the detectable substance.

In measuring the glycated protein by an enzymatic method, the protease serves the role of producing the substrate of the color reaction. Therefore, a sufficient amount of the protease is required for supplying a sufficient amount of substrate in a predetermined period. However, it is not only the glycated protein that is decomposed by the protease, and other enzymes required for the assay (for example, oxidase) are also decomposed simultaneously with the glycatedprotein, and therefore, presence of the protease at a high concentration results in poor assay precision of the glycated protein that is the target of the measurement.

In the meanwhile, hydrogen peroxide is generally measured using a Trinder's reagent which develops color by oxidative condensation between a coupler such as 4-aminoantipyrine (4-AA) or 3-methyl-2-benzothiazolinonehydrazone (MBTH) and a phenol, aniline, or toluidine chromogen in the presence of peroxidase (POD) ; or a leuco dye which directly develops color in the presence of POD. Exemplary known leuco dyes include triphenylmethane leuco dyes having an improved solubility in water (See Patent Document 3), and such dye is useful in the high sensitivity measurement.

[Patent Document 1] JP-A-5-192193
[Patent Document 2] JP-A-2001-95598
[Patent Document 3] JP-A-3-206896

### Disclosure of the Invention

#### Problems to be Solved by the Invention

In view of the situation as described above, an object of the present invention is to provide a method for measuring a glycated protein, a glycated peptide, or a glycated amino acid wherein proteolytic activity of the protease is controlled to thereby realize high accuracy of the measurement. Another object of the present invention is to provide a reagent which is used in such measurement.

#### Means for Solving the Problems

The inventors of the present invention made an intensive study in view of such situation and found that, in enzymatically measuring the glycated protein, proteolytic activity of the protease can be controlled to enable measurement of the glycated protein and the like at a high accuracy by adjusting the reaction solution before reacting with the glycated peptide-specific enzyme or the glycated amino acid-specific enzyme to a pH of 1 to 5. The present invention has been completed on the bases of such findings.

Accordingly, this invention provides a method of measuring a glycatedprotein, a glycatedpeptide, or a glycated amino acid comprising the steps of treating a sample containing the glycated protein with a protease for releasing a glycated peptide or a glycated amino acid; reacting the released glycated peptide or glycated amino acid with corresponding oxidase for generation of hydrogen peroxide; and measuring the resulting hydrogen peroxide with peroxidase and oxidizable color developing reagent; wherein reaction solution before the reaction with the oxidase is adjusted to a pH of 1 to 5.

This invention also provides a reagent for measuring a glycated protein, a glycatedpeptide, or a glycated amino acid at least containing (1) an oxidase which reacts with the glycated peptide or the glycated amino acid to produce hydrogen peroxide, (2) a solution for adjusting the reaction solution to a pH of 1 to 5, and (3) peroxidase.

Effect of the Invention

According to the present invention, a glycatedprotein, a glycated peptide, or a glycated amino acid can be measured at a high accuracy by controlling the proteolytic activity of the protease, and because of the convenience of the procedure, the method of the present invention is quite useful in the field of clinical examination.

<u>Brief Description of the Invention</u>

FIG. 1 is a view showing the results of the hemoglobin concentration measurement when Triton X-100 was added to acidic reagent (Example 3).
FIG. 2 is a view showing the results of the hemoglobin concentration measurement when EMAL 20C was added to acidic reagent (Example 3).
FIG. 3 is a view showing the results of the hemoglobin concentration measurement when a surfactant was not added to the acidic reagent (Comparative Example 3).
FIG. 4 is a view showing the correlation between the HbA1c value of the present invention and the HbA1c value measured by "Rapidia HbA1c" (Example 4).
FIG. 5 is a view showing the correlation between the HbA1c value of the present invention and the HbA1c value measured by "Rapidia HbA1c" (Example 4).
FIG. 6 is a view showing the correlation between the HbA1c value of the present invention and the HbA1c value measured by "Rapidia HbA1c" (Example 5).
FIG. 7 is a view showing the correlation between the HbA1c value of the present invention and the HbA1c value measured by "Rapidia HbA1c" (Example 6).

<u>Best Mode for Carrying Out the Invention</u>

As described above, the glycated protein in the present invention may be any glycated protein produced by unenzymatic binding between a protein and an aldose such as glucose. Exemplary glycated proteins of biological sample include glycated albumin and glycated hemoglobin, and use of the present invention facilitates measurement of, for example, hemoglobin Alc (HbAlc).

Examples of the sample containing glycated protein include biological samples such as whole blood, blood cell, serum, plasma, spinal fluid, sweat, urine, lachrymal fluid, saliva, skin, mucosa, and hair, etc. Glycated proteins are also contained in a wide variety of foods such as juice and sauce, etc. Of such samples, whole blood, blood cell, serum, and plasma are preferred, and these samples may be used in the assay with no further processing, or after a treatment such as filtration or dialysis, and in some cases, after concentrating or extracting the glycatedprotein to be measured and optionally diluting with water or a buffer solution.

In the present invention, the sample containing a glycatedprotein is allowed to react with a protease for release of a glycated peptide (for example, a fructosyl peptide) or a glycated amino acid (for example, a fructosyl amino acid). In the case of a biological sample or a food, fructosyl peptides or fructosyl amino acids generated by binding of glucose to the peptide or the amino acid formed by proteolysis of the glycated protein and the subsequent Amadori rearrangement are already present in the sample before the treatment with a protease, and such fructosyl peptides and fructosyl amino acids are also included in the "released fructosyl peptides or fructosyl amino acids".

The protease used is not particularly limited as long as it has proteolytic or peptidolytic activity. However, the protease used preferably is the one capable of releasing the fructosyl peptide or the fructosyl amino acid from the glycated protein in a short time and at a high efficiency. In particular, when the glycated protein is HbA1c, the protease used is preferably the one releasing fructosyl valyl peptide or fructosyl valyl histidine, and the most preferred is the one releasing fructosyl valyl histidine.

Examples of the protease which releases the fructosyl peptide or the fructosyl amino acid include those derived from a microorganism such as Bacillus sp., Aspergillus sp., or Streptomyces sp.; an animal; or a plant. The protease may also be the one belonging to metalloproteinase, neutral protease or basic protease, or the one produced by genetic engineering of the genes included in the microorganism. If desired, the protease may also be chemically modified.

Exemplary such proteases include those readily available as a commercial product for research purpose such as proteinase, trypsin, papain, and pronase; and those available as a commercial product for industrial purpose such as Neutral protease and Toyozyme NEP (both are products of Toyobo Co., Ltd.), Sumizyme LP, Sumizyme FP, and Sumizyme MP (these three are products of Shin Nihon Chemical Co., Ltd), Thermoase P, Protin A, and Protin P (these three are products of Daiwa Kasei K. K.), Actinase AS, Actinase PF, andActinase E (these three are products of Kaken Pharmaceutical Co. , Ltd.), and Umamizyme, Protease S "Amano" G, Protease A "Amano" G, and Protease P "Amano" 3G (these four are products of Amano Enzyme Inc.). Such protease may be used alone or in a combination of two or more.

Among such proteases, the most preferred are those derived from Streptomyces griseus since such protease can release the fructosyl valyl histidine at a high efficiency by using the protease alone. Examples of the protease derived from Streptomyces griseus include Actinase AS, Actinase AF, and Actinase E (these three product of Kaken Pharmaceutical Co., Ltd.) and Pronase E (product of Calbiochem-Novabiochem or Sigma). Also preferred are proteases derived from a Bacillus sp. and examples include Protin PC10F (product of Daiwa Kasei K.K.), and Toyozyme (product of Toyobo Co., Ltd.).

The protease as described above is preferably the one having an optimal pH of 5. 5 to 10, namely, the one having a proteolytic activity at pH 1 to 5 which is lower than the proteolytic activity at pH 5.5 to 10. The protease activity can be confirmed by a method using casein for the substrate, or by reacting the protease with a glycated peptide or the like, and comparing the samples before and after such reaction by capillary electrophoresis.

The conditions used in treating the sample are not particularly limited as long as the glycated peptide or the glycated amino acid can be released at a high efficiency in a short time by the action of the protease on the glycated protein. Concentration of the protease used may be adequately selected based on the amount of the glycated protein in the sample and the conditions used in the treatment. However, in a typical embodiment, a protease derived from Streptomyces griseus (for example, Actinase E product of Kaken Pharmaceutical Co., Ltd.) is added at a concentration of 0.0001 to 500 mg/mL, and preferably 0.001 to 300 mg/mL.

The pH in the protease treatment is not particularly limited. However, the pH may be adjusted to the optimal pH of the enzyme using an adequate pH adjusting agent such as a buffer solution, for example, to the range of 5.5 to 10. The buffer solution is not particularly limited, and exemplary buffer solutions include phosphoric acid, phthalic acid, citric acid, Tris, maleic acid, succinic acid, oxalic acid, tartaricacid, aceticacid, boricacid, andGood'sbuffersolution. The buffer solution is not particularly limited to its concentration. The concentration, however, is preferably in the range of 0.00001 to 2 mol/L, and more preferably 0.001 to 1 mol/L. The treatment is preferably conducted at a temperature of 10 to 40°C. The resulting solution may be used with no further processing, or if desired, heated, centrifuged, centrifuged, or diluted as needed.

In the measurement method of the present invention, the solution before reacting with the glycated peptide-specific enzyme or the glycated amino acid-specific enzyme (an oxidase which reacts with the glycatedpeptide or the glycated amino acid to generate hydrogen peroxide, which is hereinafter referred to as the "hydrogen peroxide-generating oxidase") is adjusted to a pH of 1 to 5, and more preferably, to the range of 1 to 4. Such adjustment of the pH to the range of 1 to 5 enables control of the proteolytic activity of the protease to the hydrogen peroxide-generating oxidase. In the present invention, "the reaction solution before reacting with the hydrogen peroxide-generating oxidase" means the reaction solution obtained by treating the sample with a protease, or a reaction solution containing both the sample solution before the treatment using the protease and the reaction solution after the treatment. In the latter case, therefore, the pH of the sample solution before the protease treatment should be adjusted to the range of 1 to 5, and the thus adjusted pH should be maintained during and after the treatment. When the sample solution is adjusted to the range of 1 to 5 before the proteolytic treatment, amount of the protease or the treatment timemaybe increased.

The agent used for adjusting the pH is not particularly limited as long as it can realize an acidic pH, and examples include inorganic acids such as hydrochloric acid, sulfuric acid, and phosphoric acid; and organic acids such as glycine, phthalic acid, maleic acid, citric acid, succinic acid, oxalic acid, tartaric acid, acetic acid, and lactic acid. The inorganic acid and the organic acid are not limited for their concentration as long as the acid can reduce the pH of the reaction solution before reacting with the hydrogen peroxide-generating oxidase to the range of 1 to 5, and the pH of the reaction solution to the range of 4 to 9 during the reaction of generating the hydrogen peroxide. Preferable concentration, however, is in the range of 0. 0001 to 1000mM.

A nonionic surfactant or an anionic surfactant each having a polyoxyethylene structure may be added to the reaction solution before reacting with the hydrogen peroxide-generating oxidase. Addition of such surfactant to the glycated protein-containing sample or the reaction solution after the protease treatment may serve as a pretreatment for collection of hemoglobin from erythrocytes for use in the reaction, or prevention of turbidity caused by the reagents or the sample.

Exemplary nonionic surfactants include polyoxyethylene alkylethers, polyoxyethylene alkylphenylethers, polyoxyethylene polyoxypropylene condensates, polyoxyethylene sorbitane fatty acid esters, polyoxyethylene fatty acid esters, and polyoxyethylene polycyclic surfactants, etc.; and the preferred are polyoxyethylene alkylphenylethers. Exemplary anionic surfactants include polyoxyethylene alkylether sulphates, polyoxyethylene alkylphenylether sulphates, polyoxyethylene alkylether phosphoric acids, polyoxyethylene alkylsulfosuccinic acids, polyoxyethylene alkylether carboxylates, and polyoxyethylene alkylether sulfonates, etc.; the preferred are polyoxyethylene alkylether phosphoric acids, polyoxyethylene alkylether sulphates, polyoxyethylene alkylsulfosuccinic acids, and polyoxyethylene alkylether sulphate; and more preferred are polyoxyethylene alkylether sulphates.

The surfactant is preferably used at an amount of 0.0001 to 10%, and most preferably at 0.001 to 10% in the reaction solution before reacting with the hydrogen peroxide-generating oxidase.

To the reaction solution adjusted to the pH of 1 to 5, oxidizable color developing reagent may be added together with peroxidase for the development of the color by the reaction with the hydrogen peroxide. In the solution having a pH of 1 to 5, the oxidizable color developing reagent is highly stable, and non-specific color development which otherwise gradually observed is suppressed. The oxidizable color developing reagent used may be any color reagent as long as it develops a color by reacting with hydrogen peroxide, and exemplary such color reagents include a combination of a coupler such as 4-aminoantipyrine and 3-methyl-2-benzothiazolinonehydrazone with an alinine compound, and leuco dye. The preferred is a leuco dye.

The leuco dye used is not particularly limited, and exemplary leuco dyes include triphenylmethane derivatives, phenothiazine derivatives, and diphenylamine derivatives, etc. Exemplary triphenylmethane derivatives include compounds having high water solubility such as those described in JP-A-3-206896 and JP-A-6-197795, etc. Exemplary phenothiazine derivatives include compounds such as those described in JP-B2-60-33479, and exemplary diphenylamine derivatives include compounds such as those described in JP-B2-60-33479, JP-A-62-93261, and the like. Among these, preferred are leucomalachite green, leucocrystal violet, sodium N-(carboxymethylaminocarbonyl)-4,4'-bis(dimethylamino)-diphenylam ine (DA-64 product of Wako Pure Chemical Industries, Ltd.), sodium 10-(carboxymethylaminocarbonyl)-3,7-bis(dimethylamino) phenothiazine (DA-67 product of Wako Pure Chemical Industries, Ltd.), 10-(N-methylcarbamoyl)-3,7-bis(dimethylamino)-10H-phenothiazine (MCDP product of Dojindo Laboratories), and N,N,N',N',N"N"hexa-(3-sulfopropyl)-4,4',4"triaminotriphenylmet hane (TPM-PS product of Dojindo Laboratories), the more preferred are TPM-PS, DA-64, DA-67, and MCDP, and the most preferred are TPM-PS and MCDP. While the leuco dyes generally have poor shelf stability in a solution, leuco dyes is stable for a prolonged time in a solution at a pH of 1 to 5.

Other leuco dyes that can be used include diaminobenzidine, hydroxyphenyl propionic acid, tetramethylbenzidine, and orthophenylenediamine.

The glycated peptide or the glycated amino acid released by the protease treatment of glycated protein can be measured by its reaction with oxidase that produces hydrogen peroxide and the subsequent measurement of the hydrogen peroxide.

The oxidase that produces hydrogen peroxide is not particularly limited as long as it can metabolyze the glycated peptide such as fructosyl peptide or the glycated amino acid such as fructosyl amino acid, and the oxidase may be the one derived from a microorganism, an animal, or a plant. If desired, the protease may also be chemically modified. Exemplary oxidases include fructosyl amino acid oxidase (JP-A-2003-79386 and WO 97/20039), ketamine oxidase (JP-A-5-192193), and fructosyl peptide oxidase (JP-A-2001-95598 and JP-A-2003-235585), and the preferred is fructosyl peptide oxidase. Examples of the fructosyl peptide oxidase include an enzyme produced by modifying fructosyl amino acid oxidase produced by Corynebacterium (JP-A-2001-95598), etc., and fructosyl peptide oxidase derived from molds (JP-A-2003-235585). The most preferred are FPOX-CE and FPOX-EE (these two are products of Kikkoman Corporation). The hydrogen peroxide-generating oxidase may be used either in the form of a solution or in dry form, may be immobilized or bonded to an insoluble carrier, and may be used alone or in combination of two or more.

The hydrogen peroxide-generating oxidase may be used at an amount of 0.001 to 1000 units/mL, and most preferably at 0.1 to 500 units/mL although the amount may vary by the type of the enzyme. In the action of the oxidase, the pH is adjusted to the range of 4 to 9 by using a buffer by considering optimal pH of the enzyme into consideration. The temperature used for the action of the oxidase is the temperature commonly used for an enzymatic reaction, and preferably in the range of 10 to 40°C. The buffer used may be selected from those described above. Although the buffer is not limited for its concentration, the concentration is preferably in the range of 0.00001 to 2 mol/L, and most preferably in the range of 0.001 to 1 mol/L.

If desired, the oxidase as described above may be used in combination with other enzymes, coenzymes, and the like. Exemplary such enzymes include amino acid metabolyzing enzymes which do not use diaphorase or fructosyl valine for the substrate, as well as enzymes such as ascorbate oxidase and bilirubinoxidase which can treat contaminant components in the blood. Exemplary coenzymes include nicotinamide adenine dinucleotide (NAD), reduced nicotinamide adenine dinucleotide (NADH), nicotinamide adenine dinucleotide phosphate (NADP), reduced nicotinamide adenine dinucleotide phosphoric acid (NADPH), thio-NAD, and thio-NADP, etc.

The peroxidase used is preferably the one derived from horseradish, and such peroxidase is preferably used at a concentration of 0.01 to 100 units/mL.

Hydrogen peroxide can be measured conveniently in a short time by an enzymatic method using a peroxidase and a reagent which develops color by oxidation. Measurement of the hydrogen peroxide is typically conducted subsequent to the generation of the hydrogen peroxide by the action of the hydrogen peroxide-generating oxidase, and in such a case, the solution used for the hydrogen peroxide measurement is preferably adjusted to pH 4 to 9 using the buffer solution as described above. The extent of the color development (change in the absorbance) maybe measured by a spectrophotometer for comparison with the absorbance of the standard glycated peptide, glycated amino acid, or the like of the known concentration to thereby measure the glycated protein, the glycated peptide, or the glycated amino acid in the sample. The measurement may be carried out by using an automated analyzer commonly used in the art.

The reagent for measuring glycated protein of the present invention contains at least (1) an oxidase which produces hydrogen peroxide by reacting with a glycated peptide or a glycated amino acid, (2) a solution for adjusting the reaction solution to a pH of 1 to 5, and (3) peroxidase. The details of each component are as described above. The term "a solution for adjusting the reaction solution to a pH of 1 to 5" of (2) as used herein means the solution having its pH adjusted with the pH adjusting agent as described above. The term "reaction solution" means the reaction solution obtained by treating the sample with a protease, and also a reaction solution containing both the sample solution before the treatment using the protease and the reaction solution after the treatment.

The reagent for measuring glycated protein of the present invention may also include a protease. Other optional components include an enzyme for processing contaminants in the blood; a reaction adjusting agent; a stabilizer; a protein such as albumin, etc.; a salt such as sodium chloride, potassium chloride, or potassium ferrocyanide, etc.; an amino acid such as lysine, alanine, aspartic acid, or glutamic acid, etc.; a peptide, a polyamino acid, or the like; a tetrazolium salt for preventing the effect of a reducing substance; an antiseptic such as an antibiotic, sodium azide, or boric acid, etc.; and a cationic surfactant.

The reagent for measuring glycated protein of the present invention may be provided in the form of a dry product or gel in addition to the solution, and in addition to the form filled in a glass bottle or a plastic container, the reagent may also be provided by coating on or impregnating in an insoluble carrier. When the reagent is stored in the form of a solution for a long period, the reagent is preferably stored in a light-resistant container.

## EXAMPLES

Next, the present invention is described in further detail by referring to the following Examples which by no means limit the scope of the present invention.

### [Example 1] Suppression of protease

(1) Preparation of samples
Samples were prepared by dissolving protease (actinase E, product of Kaken Pharmaceutical Co., Ltd.) in purified water at a concentration of 0, 1, 5, and 10 mg/mL.
(2) Measurement

<First Reagent>
3 μM fructosyl valine
20 μM
TPM-PS(N,N,N',N',N",N"-hexa-(3-sulfopropyl)-4,4',4"-triaminotriph enylmethane, product of Dojindo Laboratories)
10mM maleic acid solution (pH 3)
<Second Reagent>
4 units/mL fructosyl peptide oxidase (FPOX-CE, product of Kikkoman Corporation)
20 units/mL POD (product of Toyobo Co., Ltd.)
200mM citric acid buffer solution (pH 6)

To 20 μL of each sample was added 240 μL of the first reagent, and the mixture was incubated at 37°C for 5 minutes. After the incubating, 80 μL of the second reagent was added, and the mixture was incubated at 37°C for 5 minutes, and then, measured for the absorbance at a wavelength of 600 nm using Hitachi Model 7150 automated analyzer. Relative values were calculated on condition that measured value of change in absorbance was 100 when protease concentration in the sample was 0 mg/mL. The results are shown in Table 1.

### [Comparative Example 1]

The procedure of Example 1 was repeated except that the pH of the maleic acid solution in the first reagent was adjusted to 7 using 0.1N sodium hydroxide solution to thereby measure the absorbance.

Table 1

| Concentration of Actinase E in the sample, mg/mL | Example 1 | Comparative Example 1 |
|---|---|---|
| 0 | 100.0 | 100.0 |
| 1 | 95.6 | 91.4 |
| 5 | 78.5 | 71.2 |
| 10 | 71.4 | 54.9 |

The data demonstrated in Table 1 confirm that the proteolysis of FPOX-CE and POD by the protease is suppressed when the first reaction is conducted at a pH of 1 to 5.

[Example 2] Measurement of glycated hemoglobin

<Hemolytic reagent>
2% EMAL 20C* (product of Kao Corporation)
1 mg/mL Actinase E (product of Kaken Pharmaceutical Co., Ltd.)
20mM HEPES (2-[4-(2-hydroxyethyl)-1-piperazinyl]ethanesulfonic acid) buffer solution (pH 8)
*EMAL 20C: sodium polyoxyethylene (3) lauryl ether sulfate
<Acidic reagent>
0.1% Triton X-100
20 μM TPM-PS (product of Dojindo Laboratories)
0.05% sodium azide
5mM maleic acid solution (pH 2.8)
<Enzyme reagent>
20 units/mL POD (product of Toyobo Co., Ltd.)
4 units/mL FPOX-CE (product of Kikkoman Corporation)
200mM citric acid buffer solution (pH 6)

(1) Preparation of hemolyzed sample
Using a commercially available kit "Rapidia HbA1c" (product of Fujirebio Inc.), human blood cells containing HbA1c at a known concentration was prepared for use as a sample, and to 10 μL of this sample was added 300 μL of the hemolytic reagent to thereby prepare a hemolyzed sample.

(2) Measurement
To 20 μL of the hemolyzed sample was added 240 μL of the acidic reagent, and this mixture was incubated at 37°C for 5 minutes. After measuring absorbance at a wavelength of 600 nm, 80 μL of the enzyme reagent was added to this solution, and the mixture was allowed to react at 37°C for 5 minutes to thereby measure change in the absorbance at a wavelength of 600 nm. The results are shown in Table 2.

[Comparative Example 2]

The procedure of Example 2 was repeated except that the following Neutral reagent was used instead of the acidic reagent.
<Neutral reagent>
0.1% Triton X-100
20 μM TPM-PS (product of Dojindo Laboratories)
0.05% sodium azide
5mM maleic acid solution (pH 7)

Table 2

| HbA1c of human blood cell sample, % | Example 2, mOD | Comparative Example 2, mOD |
|---|---|---|
| 6.5 | 23.8 | 22.4 |
| 5.9 | 21.7 | 13.2 |
| 5.7 | 24.7 | 14.1 |
| 5.2 | 15.7 | 10.5 |
| 5.3 | 16.9 | 8.7 |
| 5.1 | 15.2 | 10.3 |
| 4.8 | 16.6 | 8.3 |
| 4.4 | 11.8 | 3.6 |
| 3.7 | 9.7 | 6.7 |
| Average | 17.3 | 10.9 |
| Correlation coefficient | 0.92 | 0.88 |

As demonstrated in Table 2, the values measured in Example 2 were higher than those measured in Comparative Example 2, and the correlation coefficient with the known concentration was also higher than that of Comparative Example 2. This indicates that the effect of the protease to other enzymes in the reagent is reduced.

[Example 3] Measurement of hemoglobin concentration

(1) Preparation of samples
To 10 μL of human blood cell solution was added 200 μL of 1% EMAL 20C for hemolysis, and the hemolyzed sample was diluted with 1% EMAL 20C solution to make 5 serial dilutions for use as samples.
(2) Measurement
To 20 μL of the sample was added 240 μL of the reagent containing 50mM citric acid buffer solution, and after incubating the mixture at 37°C for 5 minutes, absorbance at a wavelength of 600 nm was measured. The citric acid buffer solution was prepared by adjusting the pH to 3, 4, and 5, respectively, and adding 0.5% Triton X-100 or 1% EMAL 20C as the surfactant. The results are shown in FIGS. 1 and 2.

[Comparative Example 3]

The procedure of Example 3 was repeated except that the surfactant was not used. The results are shown in FIG. 3.

As demonstrated in FIGS. 1 to 3, when a surfactant was added to the citric acid buffer solution, absorbance increased in a manner dependent on the hemoglobin concentration. On the other hand, in the absence of the surfactant, absorbance corresponding to the serial dilution was not observed due to turbidity caused by the mixing of the hemolyzed sample of the human blood cell and the acidic reagent.

[Example 4] Measurement of HbA1c concentration

<Hemolytic reagent 1>
2% EMAL 20C (product of Kao Corporation)
1 mg/mL Actinase E (product of Kaken Pharmaceutical Co., Ltd.)
20mM HEPES buffer solution (pH 8)
<Hemolytic reagent 2>
2% EMAL 20C (product of Kao Corporation)
1 mg/mL Actinase E (product of Kaken Pharmaceutical Co., Ltd.)
260 μM TPM-PS (product of Dojindo Laboratories)
20mM HEPES (pH 8)
<Acidic reagent 1>
0.1% Triton X-100
20 μM TPM-PS (product of Dojindo Laboratories)
5mM maleic acid solution (pH 3)

<Acidic reagent 2>
0.1% Triton X-100
5mM maleic acid solution (pH 3)
<Enzymatic reagent>
20 units/mL POD (product of Toyobo Co., Ltd.)
3 units/mL FPOX-CE (product of Kikkoman Corporation)
200mM citric acid buffer solution (pH 6)
(1) Preparation of hemolyzed sample
To each of 10 samples of human blood cells was added 300 μL of the hemolytic reagent 1 or the hemolytic reagent 2 to produce the hemolyzed sample. In the followingmeasurement, (A) the Acidic reagent 1 was used when the hemolyzed sample was prepared using the Hemolytic reagent 1, and (B) the Acidic reagent 2 was used when the hemolyzed sample was prepared using the Hemolytic reagent 2.
(2) Measurement
To 20 μL of the hemolyzed sample was added 240 μL of the acidic reagent, and after incubating the mixture at 37°C for 5 minutes, absorbance at a wavelength of 600 nm was measured to determine the value dependent on the hemoglobin concentration (samp Hb). To this reaction solution was added 80 μL of enzymatic reagent, and the mixture was allowed to react at 37°C for 5 minutes. Change in the absorbance at a wavelength of 600 nm was measured, and the value dependent on the HbA1c concentration (samp A1) was determined. By using these with the value dependent on the hemoglobin concentration (std Hb) and the value dependent on the HbA1c concentration (std A1) obtained by using the samples having a known HbA1c concentration (%), the value of HbA1c (%) was calculated by the following formula:

$$\text{HbA1c (\%)} = \text{std HbA1} \times (\text{std Hb}/\text{std A1}) \times (\text{samp A1}/\text{samp Hb})$$

(std HbA1: HbA1c (%) of the sample having a known HbA1c concentration)
Correlation with the value of HbA1c (%) measured by a commercially available immunoassay kit "Rapidia HbA1c" (product of Fujirebio Inc.) (Reference Example) is shown in FIGS. 4 and 5.
As demonstrated in FIGS. 4 and 5, the method of the present invention showed good correlation with Rapidia HbA1c.
[Example 5] Measurement of HbA1c
<Hemolytic reagent>
2% EMAL 20C (product of Kao Corporation)
1 mg/mL Actinase E (product of Kaken Pharmaceutical Co., Ltd.)
20mM HEPES (pH 8)
<Acidic reagent >
0.1% Triton X-100
20 μM MCDP
(10-(N-methylcarbamoyl)-3,7-bis(dimethylamino)-10H-phenothiazine, product of Dojindo Laboratories)
10mM maleic acid solution (pH 3)
<Enzymatic reagent>
20 units/mL POD (product of Toyobo Co., Ltd.)
4 units/mL FPOX-CE (product of Kikkoman Corporation)
200mM citric acid buffer solution (pH 6)
(1) Preparation of hemolyzed sample
To 10 μL of human blood cell sample was added 300 μL of hemolytic reagent to prepare the hemolyzed sample.
(2) Measurement
To 10 μL of the hemolyzed sample was added 240 μL of the acidic reagent, and after incubating the mixture at 37°C for 5 minutes, absorbance at a wavelength of 600 nm was measured to determine the value dependent on the hemoglobin concentration. To the reaction solution was further added 80 μL of the enzyme reagent, and after allowing to react at 37°C for 5 minutes, change in the absorbance at a wavelength of 600 nm was measured to determine the value dependent on the HbA1c concentration. The value of HbA1c (%) was calculated as in the case of Example 4. Correlation with the value of HbA1c (%) measured by "Rapidia HbA1c" (product of Fujirebio Inc.) is shown in FIG. 6.

As demonstrated in FIG. 6, the method of the present invention showed good correlation with Rapidia HbA1c.
[Example 6] Measurement of HbA1c concentration
<Hemolytic reagent>
2% EMAL 20C (product of Kao Corporation)
10 mg/mL Protin PC10F (product of Daiwa Kasei K.K.)
20mM HEPES (pH 8)
<Acidic reagent >
0.1% Triton X-100
20 μM TPM-PS (product of Dojindo Laboratories)
10mM maleic acid solution (pH 3)
<Enzymatic reagent>
20 units/mL POD (product of Toyobo Co., Ltd.)
3 units/mL FPOX-CE (product of Kikkoman Corporation)
200mM citric acid buffer solution (pH 6)
(1) Preparation of hemolyzed sample
To 10 μL of human blood cell sample was added 300 μL of hemolytic reagent to prepare the hemolyzed sample.
(2) Measurement
To 10 μL of the hemolyzed sample was added 240 μL of the acidic reagent, and after incubating the mixture at 37°C for 5 minutes, absorbance at a wavelength of 600 nm was measured to determine the value dependent on the hemoglobin concentration. To the reaction solution was further added 80 μL of the enzyme reagent, and after allowing to react at 37°C for 5 minutes, change in the absorbance at a wavelength of 600 nm was measured to determine the value dependent on the HbA1c concentration. The value of HbA1c (%) was calculated as in the case of Example 4. Correlation with the value of HbA1c (%) measured by "Rapidia HbA1c" (product of Fujirebio Inc.) (Reference Example) is shown in FIG. 7.

As demonstrated in FIG. 7, the method of the present invention showed good correlation with Rapidia HbA1c.

[Example 7] Stability of TPM-PS (1)

TPM-PS was dissolved in the following aqueous solutions so that the resulting TPM-PS concentration was 60 μM, and after storing the solution at 37°C, the absorbance at a wavelength of 600 nm was measured. The absorbance at 0 hour, 2 weeks, and 3 weeks is shown in Table 3.
Table 3

| Aqueous solution | At 0 hour | At 2 weeks | At 3 weeks |
|---|---|---|---|
| 20mM PB-K*, pH 8 | 0.023 | 0.132 | 0.215 |
| 5mM tartaric acid, pH 2.8 | 0.024 | 0.064 | 0.088 |
| 5mM maleic acid, pH 2.5 | 0.023 | 0.050 | 0.072 |
| 5mM citric acid, pH 2.8 | 0.022 | 0.057 | 0.080 |
| *PB-K: potassium phosphate solution | | | |

As demonstrated in Table 3, nonspecific color development of the TPM-PS was suppressed in aqueous solution at a pH of 1 to 5.

[Example 8] Stability of TPM-PS (2)

TPM-PS was dissolved in each of the following aqueous solutions to the TPM-PS concentration of 100 μM,

and the solution was stored at 25°C for 10 days. The solution was then evaluated for its absorbance at a wavelength of 600 nm. The results are shown in Table 4.

Table 4

| | pH of the stock solution | Change in 10 days, OD |
|---|---|---|
| HC1-KCl | 1<br>2 | -0.01<br>0.01 |
| 100mM glycine-HCl | 2<br>3 | 0.00<br>-0.01 |
| 100mM citric acid buffer solution | 3<br>4<br>5 | -0.01<br>0.08<br>0.15 |
| 100mM potassium phosphate buffer | 6<br>7<br>8 | 0.22<br>0.16<br>0.17 |
| Control (purified water) | Not adjusted | 0.22 |

As demonstrated in Table 4, TPM-PS showed small change in absorbance in aqueous solutions at pH of 1 to 5 to indicate its stability.

[Example 9] Stability of MCDP

MCDP was dissolved in methanol to 4mM, and the solution was added to each of the following aqueous solutions containing 0.1% Triton X-100 to a MCDP concentration of 100 μM. The solution was stored at 37°C for 24 hours, and absorbance at a wavelength of 600 nm was measured. The results are shown in Table 5.

Table 5

| | pH of the stock solution | Change in 24 hours, OD |
|---|---|---|
| HCl-KCl | 1<br>2 | 0.01<br>0.01 |
| 50mM glycine-HCl | 2<br>3 | 0.00<br>0.00 |
| 50mM citric acid buffer solution | 3<br>4<br>5 | 0.01<br>0.06<br>0.12 |
| 50mM potassium phosphate buffer | 6<br>7<br>8 | 0.83<br>1.47<br>1.51 |
| Control (purified water) | Not adjusted | 0.71 |

As demonstrated in Table 5, MCDP showed small change in absorbance in aqueous solutions at pH of 1 to 5, indicating the suppression of the nonspecific color development as well as stability.

## Claims

1. A method of measuring a glycated protein, a glycated peptide, or a glycated amino acid comprising the steps of treating a sample containing the glycated protein with a protease for releasing a glycated peptide or a glycated amino acid; reacting the released glycated peptide or glycated amino acid with corresponding oxidase for generation of hydrogen peroxide; and measuring the resulting hydrogen peroxide with peroxidase and an oxidizable color developing reagent; wherein reaction solution before the reaction with the oxidase is adjusted to a pH of 1 to 5.

2. The method according to claim 1 wherein the glycated protein is a glycated hemoglobin.

3. The method according to claim 1 or 2 wherein the protease is the one produced from a microorganism of Bacillus, Aspergillus, or Streptomyces, or a microorganism produced by genetically engineering such microorganism; the protease has an optimal pH range of 5.5 to 10; and the protease is capable of releasing fructosyl valyl histidine by itself or by its use with another protease.

4. The method according to any one of claims 1 to 3 wherein the protease is the one produced from a microorganism derived from Streptomyces griseus; the protease has an optimal pH of 5.5 to 10; and the protease is capable of releasing fructosyl valyl histidine by itself.

5. The method according to any one of claims 1 to 4 wherein reaction solution before the reaction with the oxidase contains an anionic surfactant or a nonionic surfactant each having polyoxyethylene structure.

6. The method according to any one of claims 1 to 5 wherein the reaction solution adjusted to pH 1 to 5 contains an oxidizable color developing reagent.

7. The method according to claim 6 wherein the oxidizable color developing reagent is a leuco dye selected from triphenylmethane leuco dyes, phenothiazine leuco dyes, and diphenylamine leuco dyes.

8. The method according to claim 7 wherein the triphenylmethane leuco dye is N,N,N',N',N"N"-hexa-(3-sulfopropyl)-4,4',4"-triaminotriphenylmet hane.

9. The method according to claim 7 wherein the phenothiazine leuco dye is 10-(carboxymethylaminocarbonyl)-3,7-bis (dimethylamino) phenothiazine or 10-(N-methylcarbamoyl)-3,7-bis(dimethylamino)-10H-phenothiazine.

10. The method according to any one of claims 1 to 9 wherein the pH adjustment is conducted by using at least one member selected from hydrochloric acid, sulfuric acid, phosphoric acid, and organic acids.

11. The method according to claim 10 wherein the organic acid is glycine, maleic acid, or citric acid.

12. A reagent for measuring a glycated protein, a glycated peptide, or a glycated amino acid at least comprising (1) an oxidase which reacts with the glycated peptide or the glycated amino acid to produce hydrogen peroxide, (2) a solution for adjusting the reaction solution to a pH of 1 to 5, and (3) peroxidase.

Fig. 1

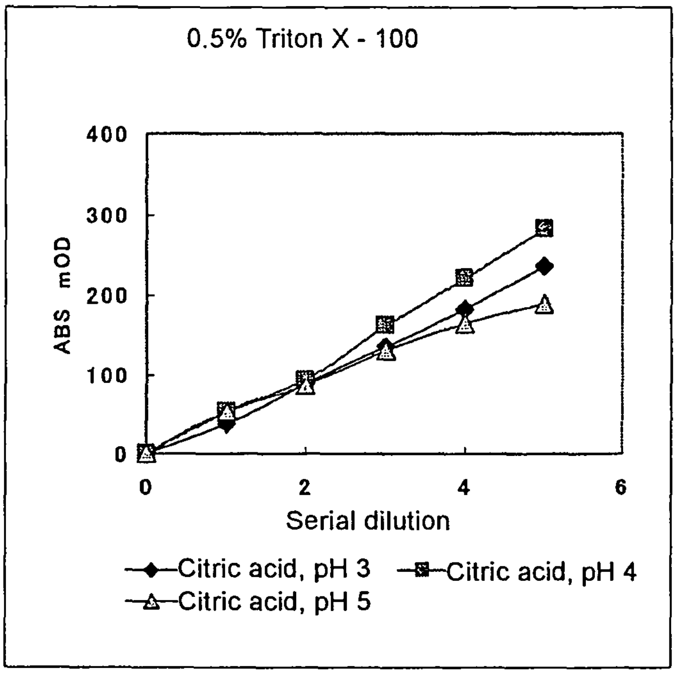
0.5% Triton X - 100
ABS mOD
400
300
200
100
0
0
2
4
6
Serial dilution
Citric acid, pH 3
Citric acid, pH 4
Citric acid, pH 5

Fig. 2

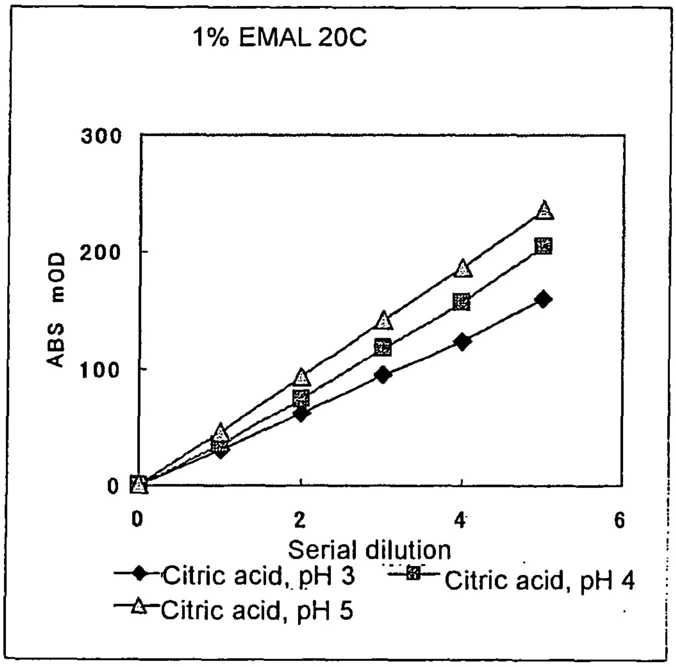
1% EMAL 20C
ABS mOD
300
200
100
0
0
2
4
6
Serial dilution
Citric acid, pH 3
Citric acid, pH 4
Citric acid, pH 5

Fig. 3

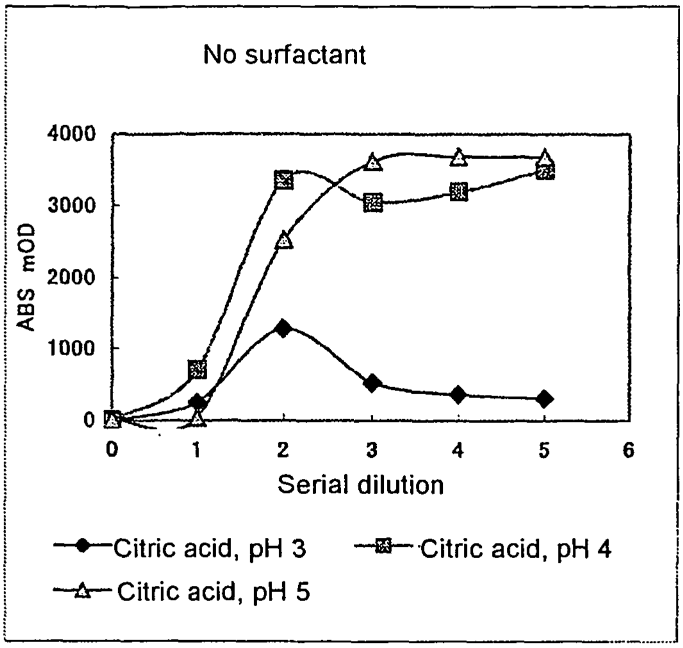
No surfactant
4000
3000
2000
1000
0
ABS mOD
0
1
2
3
4
5
6
Serial dilution
Citric acid, pH 3
Citric acid, pH 4
Citric acid, pH 5

Fig. 4

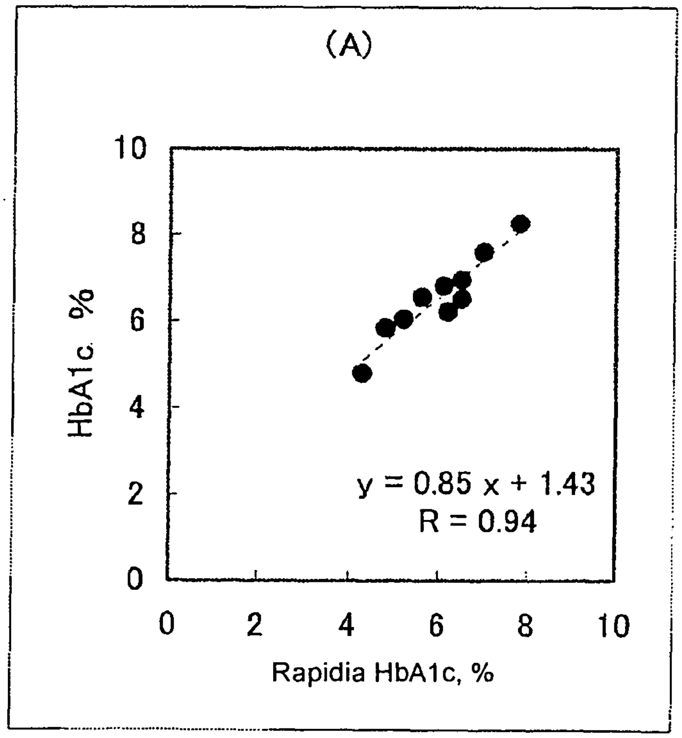
(A)
10
8
6
4
2
0
HbA1c. %
0
2
4
6
8
10
Rapidia HbA1c, %
y = 0.85 x + 1.43
R = 0.94

Fig. 5

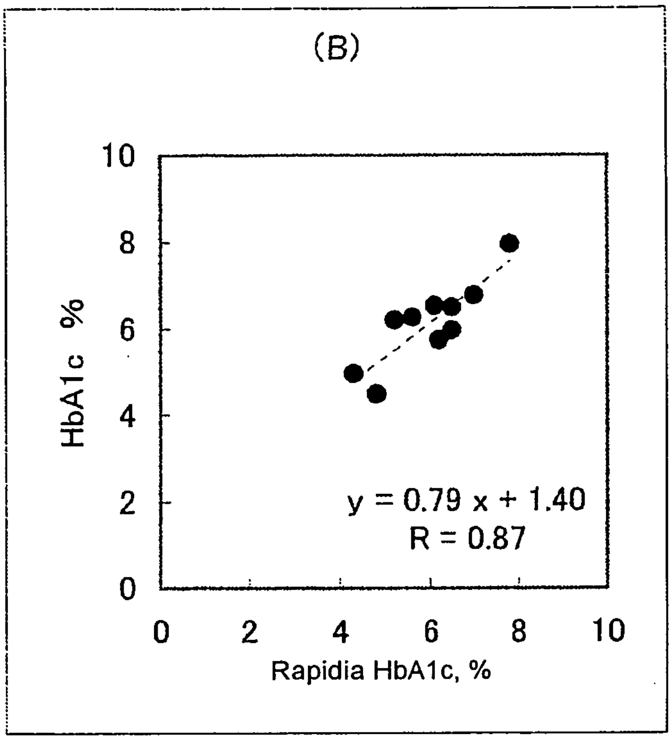
(B)
10
8
6
4
2
0
HbA1c %
0
2
4
6
8
10
Rapidia HbA1c, %
y = 0.79 x + 1.40
R = 0.87

Fig. 6

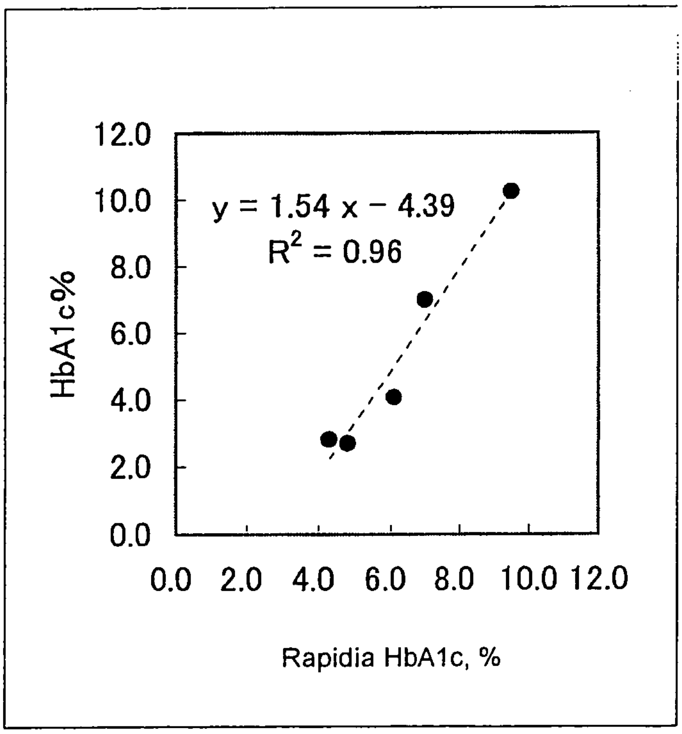
12.0
10.0
8.0
6.0
4.0
2.0
0.0
HbA1c%
0.0
2.0
4.0
6.0
8.0
10.0
12.0
Rapidia HbA1c, %
y = 1.54 x − 4.39
R2 = 0.96

Fig. 7

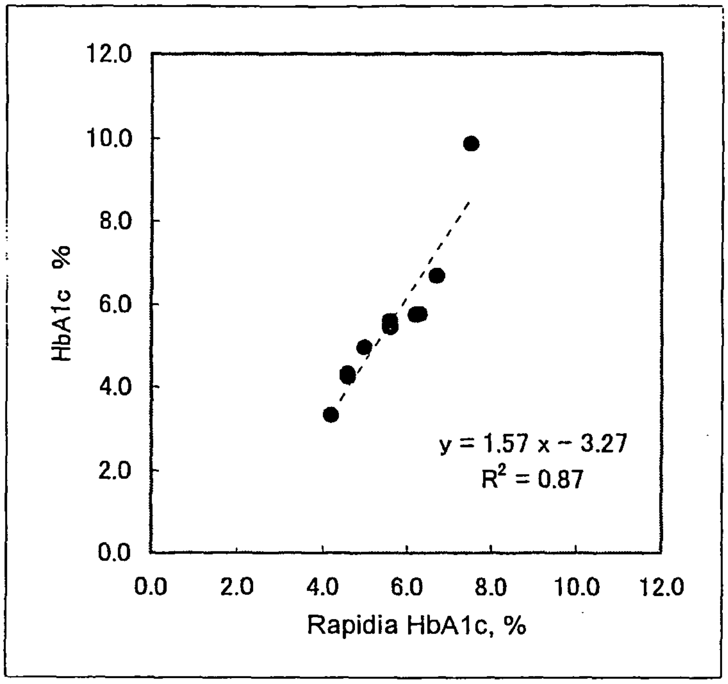
12.0
10.0
8.0
6.0
4.0
2.0
0.0
HbA1c %
y = 1.57 x - 3.27
R2 = 0.87
0.0
2.0
4.0
6.0
8.0
10.0
12.0
Rapidia HbA1c, %

## INTERNATIONAL SEARCH REPORT

International application No.
PCT/JP2005/004639

A. CLASSIFICATION OF SUBJECT MATTER
Int.Cl$^7$ C12Q1/37, 1/26, 1/28, G01N33/72

According to International Patent Classification (IPC) or to both national classification and IPC

B. FIELDS SEARCHED

Minimum documentation searched (classification system followed by classification symbols)
Int.Cl$^7$ C12Q1/37, 1/26, 1/28, G01N33/72

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
JICST FILE(JOIS), EUROPAT(QUESTEL), MEDLNIE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | JP 2001-204494 A (Koji HATADE), 31 July, 2001 (31.07.01), (Family: none) | 1-12 |
| A | JP 2001-057897 A (Daiichi Pure Chemicals Co., Ltd.), 06 March, 2001 (06.03.01), (Family: none) | 1-12 |
| A | JP 2001-095598 A (Kikkoman Corp.), 10 April, 2001 (10.04.01), & WO 2001/025475 A1 & EP 1223224 A1 | 1-12 |
| A | WO 1997/013872 A1 (Kabushiki Kaisha Kyoto DaiichiKagaku), 17 April, 1997 (17.04.97), (Family: none) | 1-12 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 April, 2005 (26.04.05) | 17 May, 2005 (17.05.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5192193 A
- JP 2001095598 A
- JP 3206896 A
- JP 6197795 A
- JP 60033479 B
- JP 62093261 A
- JP 2003079386 A
- WO 9720039 A
- JP 2003235585 A